**Europäisches Patentamt**

**European Patent Office**

**Office européen des brevets**

(19)

(11) Numéro de publication: **0 153 237**

**B1**

(12) **FASCICULE DE BREVET EUROPÉEN**

(45) Date de publication du fascicule du brevet:
12.08.87

(21) Numéro de dépôt: **85400235.9**

(22) Date de dépôt: **13.02.85**

(51) Int. Cl.⁴: **A 61 F 5/02**

ERRATUM

(SEITE, SPALTE, ZEILE)
(PAGE, COLUMN, LINE)
(PAGE, COLONNE, LIGNE)

| DIE TEXTSTELLE :<br>TEXT PUBLISHED :<br>LE PASSAGE SUIVANT : | | | | LAUTET BERICHTIGT:<br>SHOULD READ :<br>DEVRAIT ETRE LU : |
|---|---|---|---|---|
| des bandes de jointure elastique 12, 13, 14 et 15 qui, | 5 | .8 | 61/62 | des bandes de jointure elastique 13, 14, 15 et 16 qui, |
| l'élément sternal commun 33 et à l'arrière | 6<br>7 | 10<br>11 | 65-<br>01 | l'élément sternal commun 23 et à l'arrière |

| Tag der Entscheidung über die Berichtigung<br>Date of decision on rectification:<br>Date de décision portant sur modification: | )<br>)<br>)<br>)<br>) | 02.10.87<br>................. | Ausgabe- und Ver-öffentlichungstag:<br>Issue and publication date:<br>Date d'edition et de publication: | )<br>)<br>)<br>)<br>) | 11.05.88<br>................. | Patbl.Nr)<br>EPB no:)........<br>Bull. no:) | 88/19 |

**Europäisches Patentamt**

**European Patent Office**

**Office européen des brevets**

(19)

(11) Numéro de publication: **0 153 237**
**B1**

(12) # FASCICULE DE BREVET EUROPÉEN

(45) Date de publication du fascicule du brevet:
**12.08.87**

(21) Numéro de dépôt: **85400235.9**

(22) Date de dépôt: **13.02.85**

(51) Int. Cl.⁴: **A 61 F 5/02**

(54) **Appareillage externe du tronc.**

(30) Priorité: **14.02.84 FR 8402202**

(43) Date de publication de la demande:
**28.08.85 Bulletin 85/35**

(45) Mention de la délivrance du brevet:
**12.08.87 Bulletin 87/33**

(84) Etats contractants désignés:
**AT BE CH DE GB IT LI LU NL SE**

(56) Documents cité:
**FR-A-2 085 624**
**US-A-3 282 264**
**US-A-3 543 748**

(73) Titulaire: **Salort, Guy, 219, rue Raymond Losserand, F-75014 Paris (FR)**

(72) Inventeur: **Salort, Guy, 219, rue Raymond Losserand, F-75014 Paris (FR)**

(74) Mandataire: **Peuscet, Jacques, Cabinet Peuscet 68, rue d'Hauteville, F-75010 Paris (FR)**

EP 0 153 237 B1

## Description

La présente invention a trait à un appareillage externe du tronc.

Pour appareiller des handicapés moteurs du tronc, on utilise actuellement des dispositifs de trois types: un premier type (voir FR-A- 2 085 624) comporte essentiellement une structure rigide ou semi-rigide enveloppant le tronc, telle qu'une coque en matière plastique ou un corset métallique, en cuir ou composite, en tissu et acier; un second type (voir US-A- 3 282 264) comporte une potence postérieure et/ou latérale coopérant avec un corset pour supporter l'utilisateur, et est généralement appelé "corset lyonnais", tandis que le troisième type (voir US-A- 3 543 748) comprend les corsets élastiques enserrant le tronc de façon homogène.

Ces appareillages connus ne remplissent que très imparfaitement, voire pas du tout, les fonctions de réactivation des unités motrices du tronc de l'utilisateur, et donc les fonctions de récupération de ce dernier, et ils ne lui permettent pas de recouvrer la sensibilité du tronc si celle-ci a été perdue.

Par la présente invention, on se propose de réaliser un appareillage qui remédie, dans une mesure significative, aux inconvénients présentés ci-dessus.

Plus précisément, le but de la présente invention est de proposer un tel appareillage ou orthèse, qui permette de compenser plus ou moins complètement tous les troubles déficitaires du tonus et/ou de la motricité et/ou de la sensibilité aux niveaux de la colonne vertébrale et/ou du tronc d'un sujet.

Le principe, qui est à la base de l'invention, consiste à réaliser un appareillage capable d'encaisser les variations différentielles des diamètres du tronc, qui résultent des mouvements respiratoires ou d'autres mouvements volontaires du tronc ou de parties de celui-ci, et capable de les restituer et de les transmettre de façon dynamique à l'intérieur du système pratiquement conservatif, sur le plan énergétique, que l'appareillage constitue avec le tronc de l'utilisateur appareillé, en donnant des appuis dynamiques aux muscles de la respiration, de la statique et de la motricité.

Selon l'invention, ceci est assuré au moyen d'un appareillage autonome constitué par une structure composite, amovible et légère, réalisant une liaison privilégiée entre les moteurs scapulaires et le moteur pelvien de l'utilisateur ainsi appareillé. De ce fait, l'appareillage selon l'invention peut être avantageusement combiné à un appareillage externe pour handicapés moteurs d'au moins un membre supérieur et/ou à un appareillage externe pour handicapés moteurs d'au moins un membre inférieur qui fait l'objet de la demande de brevet EP-A- 0 066 028. En associant l'appareillage selon l'invention aux para-squelettes externes des membres supérieurs et inférieurs décrits dans les demandes de brevet ci-dessus référencées, il est possible de constituer un appareillage concernant l'ensemble de l'appareil locomoteur du sujet équipé. Il faut également noter que l'appareillage selon l'invention permet d'appliquer, au niveau du tronc, les principes biomécaniques sur la base desquels ont été développés les appareillages du train antérieur et du train porteur respectivement décrits dans les deux demandes de brevet précédemment mentionnées.

A cet effet, l'appareillage externe du tronc selon la présente invention se caractérise par le fait qu'il comprend deux structures composites,

- la première structure étant une structure inférieure, de forme générale externe tronconique, destinée à enserrer le bassin et l'abdomen et constituée par un corset pelvien s'étendant sensiblement du périmètre bi-trochantérien en bas jusqu'à, d'une part, sensiblement l'ombilic, en haut et en avant, et, d'autre part, la moitié inférieure de la colonne lombaire, en haut et en arrière, ledit corset comportant:

a) une pluralité d'éléments semi-rigides reliés par des organes souples et flexibles;

b) des moyens élastiques régulateurs, reliant chacune des deux parties du corset situées au droit des deux épines iliaques antéro-supérieures à la partie du corset située au droit de l'attache sacro-iliaque homo-latérale, d'une part, et à la partie du corset située au droit de l'ischion homo-latéral, d'autre part;

- la deuxième structure composite étant une structure supérieure comportant deux enveloppes hémithoraciques symétriques, qui ont chacune une forme générale externe tronconique et entourent chacune un hémi-thorax, les deux enveloppes hémi-thoraciques présentant une partie sterno-xyphoïdienne commune et comportant chacune deux sous-ensemblés, dont l'un est un sous-ensemble scapulaire et l'autre un sous-ensemble thoracique inféro-latéral, chacun de ces deux sous-ensembles étant constitué d'une pluralité d'éléments semi-rigides reliés par des organes souples et flexibles, les régions du creux axillaire et du sein restant libres, la structure supérieure comportant également:

a) une articulation élastique dorsale, centrée au sommet de la colonne dorsale et reliant l'une à l'autre, et de manière détachable, les deux enveloppes hémi-thoraciques;

b) des moyens élastiques régulateurs reliant, sur chacune des deux enveloppes hémi-thoraciques, d'une part, les éléments semi-rigides du sous-ensemble scapulaire, et d'autre part, les éléments semi-rigides du sous-ensemble thoracique;

les deux structures composites précitées étant reliées l'une à l'autre par des moyens de liaison et de réglage de l'équilibre dynamique de l'appareillage, qui comprennent:

a) deux leviers-ressorts antérieurs, tendus chacun entre l'une des deux parties du corset située au droit des épines iliaques antéro-supérieures et la portion homolatérale de la partie sterno-xyphoïdienne commune, lesdits leviers-ressorts étant capables d'encaisser les déformations en flexion et/ou en torsion du tronc et de régler les mouvements relatifs de la colonne vertébrale, du bassin et de l'ensemble thoracique;

b) deux tendeurs latéro-postérieurs reliant chacun la zone sous-épineuse du sous-ensemble scapulaire de l'une des deux enveloppes hémi-thoraciques à la partie du corset située au droit de l'épine iliaque du côté opposé, lesdits tendeurs étant destinés à freiner une chute du thorax vers l'avant.

---------------------------

---------------------------

---------------------------

---------------------------

Dans un mode préféré de réalisation, le corset comporte quatre éléments semi-rigides, les deux premiers éléments étant sensiblement égaux et ayant approximativement la forme d'un triangle équilatéral dont le côté est sensiblement égal au demi-périmètre de la taille, l'un desdits premiers éléments étant un élément postérieur, qui se place sur la région sacro-coccygienne, de façon que son côté inférieur soit disposé selon le demi-périmètre postérieur bi-trochantérien et l'autre desdits premiers éléments étant un élément antérieur, qui se place sur la symphyse pubienne, de façon que son côté supérieur soit disposé selon le demi-périmètre antérieur de la taille, les deux autres éléments semi-rigides du corset étant des éléments sensiblement égaux, de forme sensiblement trapézoïdale, situés chacun en position intermédiaire entre les premiers éléments antérieur et postérieur, les côtés supérieurs de ces éléments trapézoïdaux étant disposés au niveau de la taille et étant sensiblement égaux au demi-périmètre de ladite taille, ces deux éléments intermédiaires se plaçant symétriquement de chaque côté sur les deux os iliaques.

Dans un mode préféré de réalisation, chacun des deux sous-ensembles de la deuxième structure composite est constitué de trois éléments semi-rigides reliés par des organes souples et flexibles, lesdits trois éléments semi-rigides étant, pour le sous-ensemble scapulaire, un élément pectoral, un élément trapézoïdal et un élément sous-épineux, et, pour le sous-ensemble thoracique, un élément thoracique postérieur se plaçant sur la région sous-scapulaire, un élément thoracique antérieur se plaçant sur la région sous-mammaire, et un élément xiphoïdien qui se place sur les cartilages costaux et appartient à la partie sterno-xyphoïdienne commune, les deux enveloppes hémi-thoraciques étant reliées entre elles par l'articulation élastique dorsale au niveau des éléments sous-épineux de leurs sous-ensembles scapulaires.

Ainsi, l'extrémité inférieure du tronc, c'est-à-dire le moteur pelvien, qui constitue la fondation dynamique de la colonne vertébrale, et les extrémités supérieures du tronc, c'est-à-dire les deux moteurs scapulaires supportés par la colonne dorso-lombaire, sont entourées chacune par une enveloppe tronconique et composite, dont le jeu et les déformations différentielles sont régulés par la charge des leviers-ressorts antérieurs, des tendeurs latéro-postérieurs et des moyens élastiques régulateurs du corset et des enveloppes hémi-thoraciques.

L'appareillage selon l'invention autorise ainsi tous les mouvements physiologiques de la colonne vertébrale. Pour un réglage moyen des leviers ressorts des tendeurs et des moyens élastiques régulateurs, la flexion antérieure globale de la poutre vertébrale dorso-lombaire peut atteindre 30°, et son inclinaison latérale peut atteindre 15°, tandis qu'une extension costale latérale et supérieure peut être obtenue, ainsi que toutes les combinaisons possibles de mouvements de déformations en vis (torsion-extension).

Afin de faciliter la mise en place et la fixation du corset sur l'utilisateur, le corset s'ouvre et se ferme avantageusement par l'avant, et à cet effet, l'élément semi-rigide antérieur du corset est subdivisé, le long d'une ligne verticale et médiane, en deux moitiés portant des moyens complémentaires d'un mécanisme d'ouverture et de fermeture des deux moitiés l'une par rapport à l'autre.

Dans le but de faciliter les mouvements du moteur pelvien en lordose comme en cyphose, les deux sommets adjacents audit côté horizontal des éléments triangulaires antérieur et postérieur du corset sont coupés, en délimitant des espaces libres autorisant les mouvements du bassin.

Afin de maintenir convenablement en position les quatre éléments semi-rigides du corset, qui tendent à s'écarter les uns des autres sous l'effet des mouvements du moteur pelvien, chacun des quatre éléments semi-rigides du corset est relié à chacun des deux éléments qui lui sont adjacents par une bande de jointure élastique qui occupe également les espaces éventuellement libérés par la coupe des sommets des éléments antérieur et postérieur.

Dans ce cas, il est avantageux que les parties du corset sur lesquelles sont implantés les leviers-ressorts antérieurs, les tendeurs latéro-postérieurs, et les moyens élastiques régulateurs, aux niveaux des épines iliaques, sont situées sur les bandes de jointure élastique reliant l'élément antérieur et les éléments intermédiaires, ainsi que sur les bords adjacents de ces éléments.

De même, les parties du corset sur lesquelles sont implantés les moyens élastiques régulateurs, aux niveaux des attaches sacro-iliaques et des ischions, sont situées sur les bandes de jointure élastique entre l'élément postérieur et les éléments intermédiaires ainsi que sur les bords adjacents de ces éléments.

Dans un mode de réalisation simple et pratique, les quatre éléments semi-rigides du corset sont des plaques moulées en matière plastique implantées sur une bande élastique, et les moyens élastiques régulateurs du corset sont constitués par quatre bandes élastiques régulant l'écartement des éléments intermédiaires du corset, et dont les deux bandes inférieures, reliant les parties au droit des épines iliaques aux parties au droit des ischions, forment des freins limitant le jeu antérieur de l'élément postérieur du corset et s'opposant à la lordose, tandis que les deux bandes supérieures reliant les parties au droit des épines iliaques aux parties au droit des attaches sacro-iliaques forment des freins s'opposant à la cyphose. De manière analogue, les deux tendeurs latéro-postérieurs sont chacun constitué par une bande élastique; de plus, chacun des deux leviers-ressorts est avantageusement constitué par une lame métallique flexible, capable d'encaisser et de restituer les contraintes de flexion et de torsion.

De manière avantageuse, sur le sous-ensemble scapulaire de chaque enveloppe hémi-thoracique, les éléments pectoral, trapézoïdal et sous-épineux sont des triangles équilatéraux dont le côté est approximativement égal au bord externe de l'omoplate et à la hauteur d'un élément sternal semi-rigide, de forme sensiblement rectangulaire, de la partie sterno-xyphoïdienne commune, l'élément trapézoïdal présentant un sommet dirigé vers l'épaule et le côté opposé tourné vers le cou, en étant relié, le long de son côté postérieur, au côté supérieur de l'élément sous-épineux par une bande de jointure élastique et en faisant avec lui un angle d'environ 15°, l'élément sous-épineux étant relié le long de son côté interne à l'articulation élastique dorsale et, le long de son côté externe, et par une bande de jointure élastique, à l'élément thoracique postérieur du sous-ensemble thoracique homo-latéral, en étant fixé par son sommet inférieur à l'extrémité supérieure du tendeur latéro-postérieur homo-latéral, l'élément trapézoïdal étant également relié, le long de son côté antérieur, et par une bande de jointure élastique, au côté externe de l'élément pectoral, en faisant avec lui un angle d'environ 15°, et l'élément pectoral ayant son côté supérieur tourné vers la clavicule et étant incliné d'environ 30° par rapport à l'élément sternal, à l'extrémité supérieure duquel l'élément pectoral est relié par son sommet opposé à son côté externe. De manière analogue, sur le sous-ensemble thoracique de chaque enveloppe hémi-thoracique, qui est tendu entre l'élément sous-épineux du sous-ensemble scapulaire homo-latéral et la partie sterno-xyphoïdienne commune, les éléments thoracique postérieur, thoracique antérieur et xyphoïdien sont des triangles équilatéraux présentant un sommet dirigé vers le bas, l'élément thoracique antérieur étant relié d'une part le long de son côté interne au côté externe de l'élément xyphoïdien par une bande de jointure élastique, et, d'autre part, le long de son côté externe au côté externe de l'élément thoracique postérieur par une bande de jointure élastique, en faisant entre ces côtés respectifs des angles d'environ 60°. Il est possible que la partie sterno-xyphoïdienne commune soit constituée d'une seule pièce présentant l'élément sternal et les deux éléments xyphoïdiens. Mais, de préférence, chacun des deux éléments xyphoïdiens triangulaires est relié par son sommet interne supérieur à l'extrémité inférieure de l'élément sternal.

Dans une forme de réalisation simple et pratique, les trois éléments du sous-ensemble scapulaire et les trois éléments du sous-ensemble thoracique sont, pour chaque enveloppe hémi-thoracique, formés par une chaîne de six plaques en triangle équilatéral moulées en matière plastique et implantées sur une bande élastique. Avantageusement, les deux leviers-ressorts antérieurs sont implantés par leurs extrémités supérieures chacun sur l'élément xyphoïdien homolatéral correspondant. De plus, les moyens élastiques régulateurs reliant les trois éléments semi-rigides du sous-ensemble scapulaire sur chacune des deux enveloppes hémi-thoraciques sont constitués par un tendeur élastique ancré par une extrémité sur le sommet inférieur de l'élément pectoral, par l'autre extrémité sur le sommet inférieur de l'élément sous-épineux, et passant par le centre de gravité de l'élément trapézoïdal. De manière analogue, les moyens élastiques régulateurs reliant les trois éléments semi-rigides du sous-ensemble thoracique sur chacune des deux enveloppes hémi-thoraciques sont constitués par un tendeur élastique ancré par une extrémité sur le sommet inférieur de l'élément xyphoïdien, par l'autre extrémité sur le sommet inférieur de l'élément thoracique postérieur, et passant par le centre de gravité de l'élément thoracique antérieur.

Dans une forme de réalisation simple et peu coûteuse, l'articulation élastique dorsale comprend deux organes élastiques de forme elliptique, ayant un grand axe sensiblement égal à la hauteur de la colonne dorsale et un petit axe sensiblement égal au côté des éléments en triangle équilatéral des sous-ensembles thoraciques, l'un des organes ayant la forme d'un anneau elliptique avec un grand évidement central, tandis que l'autre organe a la forme d'un disque elliptique percé d'un petit évidement central et présentant une encoche axiale s'étendant de chacune des deux extrémités de son grand axe vers son centre, et dont la profondeur est légèrement supérieure à la largeur de l'anneau elliptique aux niveaux des extrémités du grand axe de ce dernier, le disque étant engagé dans l'anneau et monté pivotant par rapport à ce dernier en chevauchant par ses encoches les extrémités du grand axe de l'anneau elliptique, de sorte que les bords internes des éléments sous-épineux sont retenus chacun dans une pince constituée par la coopération d'une moitié du disque elliptique et d'une moitié de l'anneau elliptique.

Enfin, dans un but d'esthétique et afin de faciliter l'utilisation de l'appareillage, les structures inférieure et supérieure, ainsi que les leviers ressorts et les tendeurs sont noyés dans un scaphandre.

Pour mieux faire comprendre l'objet de l'invention, on va en décrire maintenant, à titre d'exemple purement illustratif et non limitatif, un mode de réalisation représenté sur le dessin annexé.

Sur ce dessin:

- la figure 1 est une vue schématique de la face antérieure de l'appareillage disposé dans un plan, grâce à une découpe de l'élément postérieur du corset;

- la figure 2 est une vue schématique de la face postérieure de l'appareillage de la figure 1, disposé dans un plan grâce à l'ouverture des deux moitiés de l'élément antérieur du corset;

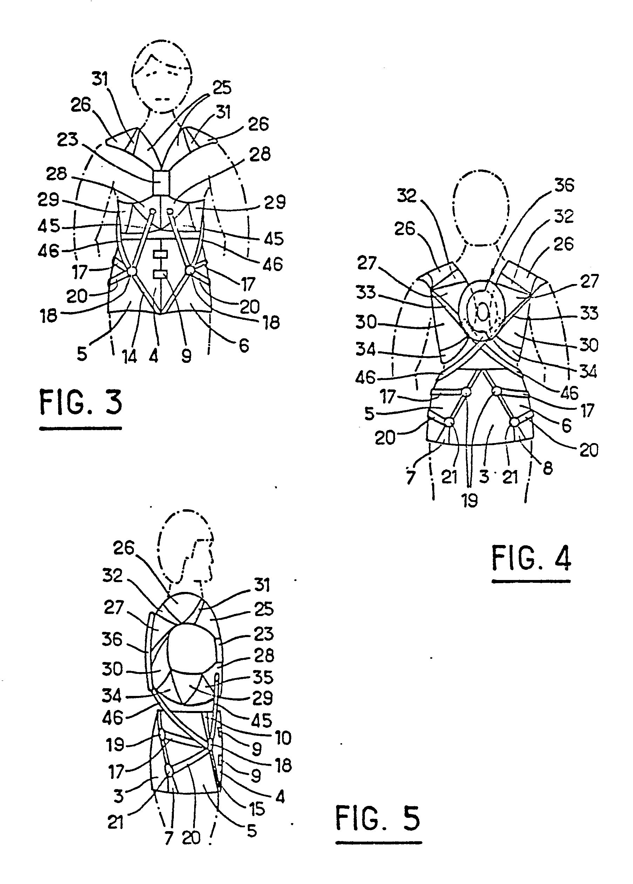

- les figures 3, 4 et 5 sont des vues schématiques de face, de dos et de profil de droite d'un utilisateur appareillé,

- les figures 6 et 7 sont des vues de face des deux organes de l'articulation élastique dorsale de l'appareillage des figures 1 à 5, et

- la figure 8 est une vue schématique en perspective montrant la coopération de cette articulation avec les deux éléments sous-épineux.

En référence aux dessins, l'appareillage comprend un corset pelvien 1 comportant essentiellement une bande élastique 2, sur laquelle sont implantées quatre plaques semi-rigides 3, 4, 5 et 6, de 2 mm d'épaisseur, moulées en matière plastique, par exemple en polyéthylène basse densité, et qui présentent une certaine souplesse en déformation dans toutes les directions. La bande élastique 2 peut avoir une forme générale rectangulaire, mais de préférence elle présente la forme d'une portion de couronne circulaire de grand rayon, de sorte que lorsque les deux petits côtés de la bande 2 sont placés en bout l'un de l'autre, la bande 2 prend la forme d'une enveloppe tronconique ayant sensiblement la forme physiologique de la partie inférieure du tronc, c'est-à-dire de la ceinture abdomino-pelvienne, que le corset pelvien 1 est destiné à enserrer. La largeur de la bande 2 est telle qu'elle s'étend du périmètre bi-trochantérien du porteur, en bas, à l'ombilic, en haut et en avant, et à la hauteur de la 3è ou de la 4è vertèbre lombaire, en haut et en arrière, c'est-à-dire au niveau de la taille. L'une des plaques 3, implantée au milieu de la bande 2, a la forme d'un triangle équilatéral dont le côté est égal au demi-périmètre postérieur de la taille du porteur. Cette plaque 3, qui constitue l'élément semi-rigide postérieur du corset 1, est disposée avec un côté horizontal inférieur le long du demi-périmètre postérieur bi-trochantérien, et le sommet opposé en pointe supérieure dirigée verticalement vers le haut. Les deux sommets adjacents au côté horizontal inférieur de la plaque 3 sont abattus par des découpes verticales en dégageant des zones triangulaires 7 et 8 de bande élastique 2. Une autre plaque 4, constituant l'élément semi-rigide antérieur du corset 1, présente également une forme de triangle équilatéral de côté égal à celui de la plaque 3, mais cette plaque 4 est implantée de manière à présenter un côté horizontal supérieur sur le demi-périmètre antérieur de la taille et le sommet opposé en pointe inférieure dirigée verticalement vers le bas, et cette plaque 4 est divisée, le long d'une ligne verticale et médiane, en deux moitiés 4a et 4b ayant chacune la forme d'un triangle rectangle, et chacune implantée sur une partie d'extrémité de la bande 2, de sorte que les deux moitiés 4a et 4b puissent être accolées pour reconstituer l'élément antérieur 4 lorsque la bande 2 est refermée en enveloppe autour de la partie inférieure du tronc du porteur. Les moitiés 4a et 4b portent les organes complémentaires d'un dispositif de fermeture et d'ouverture rapide, qui permet de solidariser les deux moitiés 4a et 4b l'une à l'autre, afin de fermer le corset pelvien 1, ou de les désolidariser l'une de l'autre afin d'ouvrir ce corset. Ce dispositif peut par exemple comporter deux paires de languettes 9 complémentaires du dispositif de fermeture disponible sous la dénomination commerciale de "Velcro", dont une languette de chaque paire, portant les crochets, est fixée sur la moitié 4a, et dont l'autre languette de chaque paire, portant les bouclettes, est fixée sur la moitié 4b, les deux languettes de la paire supérieure étant sensiblement au tiers de la largeur de la bande 2, à partir de son bord supérieur, et les deux languettes de la paire inférieure étant sensiblement au tiers de la largeur de la bande 2 à partir de son bord inférieur. Ce dispositif a l'avantage de permettre un règlage immédiat, à la taille du porteur, du corset pelvien 1 à ouverture et fermeture par l'avant qui peut s'accommoder des débordements de la masse abdominale.

Comme pour l'élément postérieur 3, l'élément antérieur 4 est amputé aux niveaux des deux sommets adjacents à son côté horizontal supérieur par deux découpes verticales (une dans chaque moitié 4a, 4b) en laissant apparaître des parties triangulaires 10 et 11 de la bande 2. Les découpes des sommets des éléments 3 et 4 autoriseront le jeu du moteur pelvien du porteur en lordose et en cyphose. Les deux autres plaques 5 et 6 sont des éléments latéraux et intermédiaires égaux, en forme de trapèze ou sensiblement de parallèlogramme, disposés symétriquement par rapport aux éléments postérieur 3 et antérieur 4, et dont l'un 5 est interposé entre la moitié 4a et l'élément postérieur 3, tandis que l'autre 6 est interposé entre la moitié 4b et l'élément postérieur 3. Les bords supérieurs sensiblement horizontaux de ces éléments intermédiaires 5 et 6 sont pratiquement égaux au demi-périmètre postérieur de la taille. Comme représenté sur les dessins, les quatre éléments 3, 4, 5 et 6 ne sont pas disposés directement adjacents les uns aux autres sur la bande 2, mais ils sont séparés par un jeu compris entre 5 et 8 mm environ, en dégageant des bandes de jointure élastique 12, 13, 14 et 15 qui, avec les zones triangulaires 7, 8, 10 et 11, constituent les seules parties apparentes de la bande élastique 2. Ces bandes de jointure élastique 13 à 16 resserrent contre le tronc du porteur les quatre éléments 3 à 6, qui recouvrent respectivement la région sacro-coccygienne, la symphyse pubienne et les deux os iliaques du porteur, et qui tendent à s'écarter les uns des autres sous l'effet des mouvements du moteur pelvien du porteur. Le corset 1 comporte également des freins propres constitués par quatre sangles élastiques règlables. Deux de ces sangles 17 relient chacune l'une des deux parties 18 du corset 1 au droit des deux épines iliaques antéro-supérieures, c'est-à-dire sensiblement à mi-hauteur le long des jointures élastiques 14 et 15, et à cheval sur ces jointures et sur les bords adjacents des éléments rigides 4, 5 et 6 correspondants, à la partie homo-latérale des deux parties 19 du corset 1 au droit des attaches sacro-iliaques, c'est-à-dire sensiblement au tiers de la hauteur le long des jointures élastiques 13 et 16 à partir du bord supérieur du corset 1, et à cheval sur ces jointures et sur les bords adjacents des éléments 3, 5 et 6 correspondants. Les deux autres sangles 20 relient chacune un point d'attache 18, au droit d'une épine iliaque, à la partie homo-latérale des deux parties 21 du corset 1 au droit des ischions, c'est-à-dire au tiers de la hauteur le long des jointures 13 et 16 à partir du bord inférieur, et à cheval sur les bords adjacents des éléments 3, 5 et 6 correspondants. Les deux sangles supérieures 17 constituent des freins s'opposant à la déformation du corset pelvien 1 en cyphose, en s'opposant au jeu antérieur de l'élément postérieur 3, tandis que les deux sangles inférieures 20 constituent des freins s'opposant à la déformation du corset 1 en lordose, l'ensemble des quatre sangles 17 et 20 s'opposant à l'écartement des deux éléments latéraux intermédiaires 5 et 6.

Au-dessus de sa structure inférieure, constituée par le corset 1, l'appareillage comprend également une structure supérieure constituée de deux enveloppes thoraciques 22a et 22b, entourant chacune un hémi-thorax, et symétriques par rapport à un élément sternal 23, de forme rectangulaire, disposé verticalement, et constitué également par une plaque semi-rigide de matière plastique moulée. Chacune de ces deux enveloppes 22a et 22b, de forme générale tronconique, est constituée par une chaîne de six plaques semi-rigides, également moulées en matière plastique, et implantées sur une bande élastique 24a, 24b. Ces six plaques, par exemple en polyéthylène basse densité de 2 mm d'épaisseur, ont la forme de triangles équilatéraux égaux, dont le côté est approximativement égal au bord externe de l'omoplate du porteur, ainsi qu'à la hauteur de l'élément sternal 23. Chaque chaîne se subdivise en deux sous-ensembles comportant chacun trois plaques, et qui sont, l'un, un sous-ensemble scapulaire ou épaulière présentant, de l'intérieur vers l'extérieur, un élément pectoral 25, un élément trapézoïdal 26 et un élément sous-épineux 27, et l'autre, un sous-ensemble thoracique inféro-latéral présentant, de l'intérieur vers l'extérieur, un élément xyphoïdien 28, recouvrant les cartilages costaux, un élément thoracique antéro-latéral 29 recouvrant la région sous-mammaire, et un élément thoracique postéro-latéral 30 recouvrant la région sous-scapulaire (entre la 6è et la 10è côte), les régions du creux axillaire et du sein étant libres.

Comme sur le corset 1, les six éléments semi-rigides 25 à 30 de chaque chaîne ne sont pas directement adjacents sur la bande élastique correspondante, mais séparés par des morceaux de bande constituant des jointures élastiques de forme triangulaire. Sur l'épaulière, l'élément pectoral 25 est relié à l'extrémité supérieure de l'élément sternal 23 par un sommet inférieur et interne, et est incliné d'environ 30° par rapport au sternum, en présentant un côté supérieur le long de la clavicule, et en étant lié le long de son côté externe au côté antérieur de l'élément trapézoïdal 26 par une jointure élastique 31 s'ouvrant sur un secteur angulaire d'environ 15°. L'élément trapézoïdal 26 a un sommet tourné vers l'épaule et le côté opposé tourné vers le cou, et se trouve relié le long de son côté postérieur au côté intérieur et supérieur de l'élément sous-épineux 27 par une jointure élastique 32, s'ouvrant sur un secteur angulaire d'environ 15°. L'élément sous-épineux 27 présente un sommet dirigé vers l'épaule et délimité entre son côté interne et supérieur et son côté externe et inférieur, le long duquel il est relié, par une jointure élastique 33 en bande, présentant un jeu de règlage de tension d'environ 1 cm, au côté supérieur et interne de l'élément thoracique postérieur du sous-ensemble thoracique inférolatéral. Ce dernier, qui est tendu entre l'élément sous-épineux 27 et l'élément pectoral 23, est tel que ses trois éléments présentent un côté tourné vers la région mammaire et le sommet opposé dirigé vers le bas. Le côté externe de l'élément thoracique postéro-latéral 30 est relié au côté externe de l'élément thoracique antéro-latéral 29 par une jointure élastique triangulaire 34 s'étendant sur un secteur angulaire d'environ 60°. De même, l'élément thoracique antéro-latéral 29 est relié par son côté interne au côté externe de l'élément xyphoïdien 28 par une jointure élastique triangulaire 35 s'étendant sensiblement sur un secteur angulaire de 60°. Enfin, l'élément xyphoïdien 28 est fixé à l'extrémité inférieure de l'élément sternal 23 par son sommet interne supérieur et se trouve accolé par son côté interne à l'élément xyphoïdien de l'autre enveloppe hémithoracique 22a ou 22b.

Il est à noter que les deux éléments xyphoïdiens 28 symétriques et rigidement fixés à l'élément sternal 23 pourraient constituer avec ce dernier une plaque d'une seule pièce.

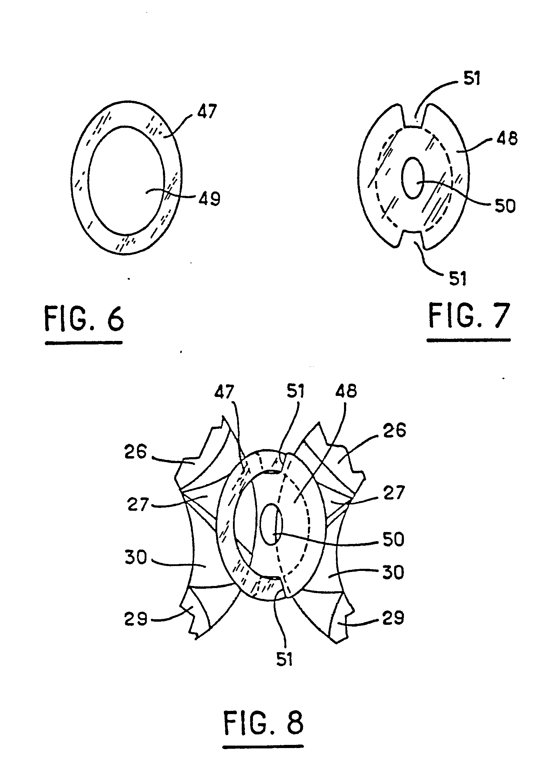

De plus, les éléments sous-épineux 27 des deux enveloppes hémi-thoraciques 22a et 22b sont reliés l'un à l'autre par leur côté interne au moyen d'une articulation dorsale élastique 36, qui sera plus précisément décrite ci-dessous en référence aux figures 6 à 8.

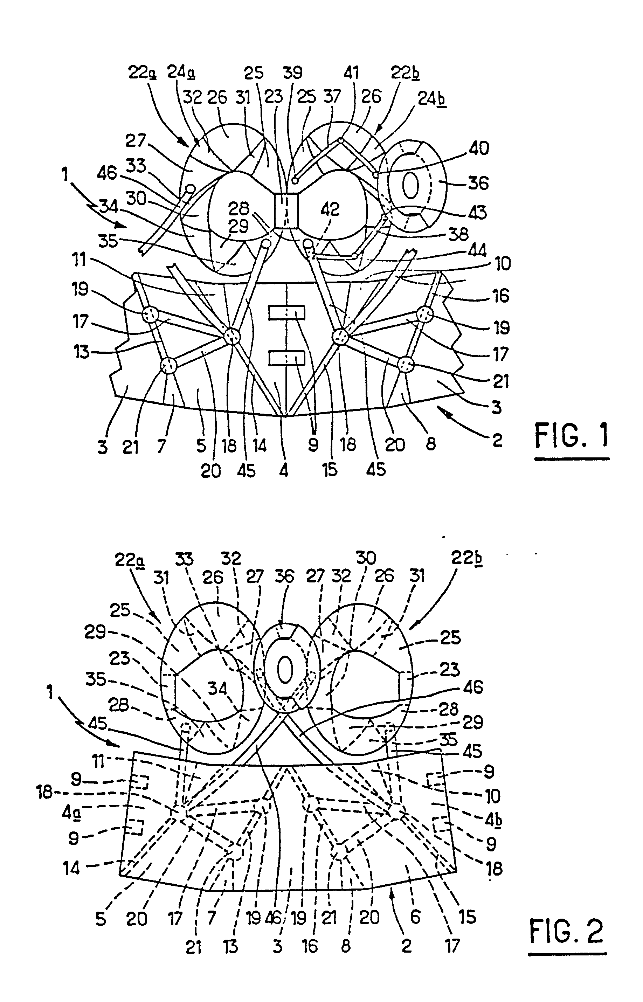

Les deux enveloppes tronconiques 22a et 22b ainsi reliées l'une à l'autre, à l'avant par l'élément sternal commun 33 et à l'arrière par l'articulation dorsale élastique 36, constituent en quelque sorte des diaphragmes dont l'ouverture et la fermeture sont régulées par deux freins 37 et 38 constitués chacun par un tendeur élastique (pour ne pas surcharger la figure 1, les tendeurs 37 et 38 n'ont été représentés que sur l'enveloppe hémi-thoracique 22b, dans la partie supérieure de droite de la figure 1). L'un des tendeurs 37 est implanté sur l'épaulière en étant ancré par l'une de ses extrémités 39 sur le sommet interne de l'élément pectoral 25 et par son autre extrémité 40 sur le sommet interne et inférieur de l'élément sous-épineux 27, en passant dans un arceau 41 fixé sur le centre de gravité de l'élément trapézoïdal 26. L'autre tendeur 38 est ancré par l'une de ses extrémités 42 sur le sommet inférieur de l'élément xyphoïdien 28, et par son autre extrémité 43 sur le sommet inférieur de l'élément thoracique postéro-latéral 30, en passant dans un arceau 44 fixé sur le centre de gravité de l'élément thoracique antéro-latéral 29.

La structure inférieure, constituée par le corset pelvien 1, et la structure supérieure, constituée par les deux enveloppes hémi-thoraciques 22a et 22b, sont reliées l'une à l'autre par des organes élastiques de réglage de l'équilibre dynamique de l'appareillage. Ces organes comprennent d'une part deux leviers-ressorts 45 antérieurs, constitués chacun par une lame d'acier trempé XC 75 de 40 mm de largeur et 0,5 mm d'épaisseur, tendue entre l'un des deux éléments xyphoïdiens 28, sur lequel elle est ancrée par son extrémité supérieure au voisinage du centre de gravité de cet élément, et le corset 1, sur lequel elle est ancrée par son extrémité inférieure au point d'attache 18 au droit de l'épine iliaque homo-latérale. Ces organes comprennent d'autre part deux tendeurs latéro-postérieurs 46, constitués chacun par une sangle élastique ancrée par son extrémité supérieure sur le sommet inférieur d'un élément sous-épineux 27 et par son extrémité inférieure sur le point d'attache 18 situé sur le corset 1 au droit de l'épine iliaque contro-latérale.

L'articulation dorsale élastique 36, qui est centrée au sommet de la colonne dorsale, a une forme générale externe elliptique, dont le grand axe s'étend verticalement sur l'ensemble de la colonne dorsale, tandis que son petit axe horizontal est approximativement égal au côté des triangles équilatéraux égaux que sont les éléments 25 à 30. Cette articulation comprend deux organes élastiques de forme elliptique 47 et 48, montés l'un dans l'autre. L'un, 47, est un anneau elliptique présentant un large évidement central 49, de forme également elliptique. L'autre, 48, est un disque elliptique ayant les mêmes grand et petit axes que l'anneau 47, mais présentant un petit orifice central 50, également de forme elliptique, et deux encoches axiales 51, ménagées chacune dans une extrémité du grand axe vers le centre, et dont la profondeur est légèrement supérieure à la largeur de l'anneau 47 dans ses parties situées aux extrémités de son grand axe, c'est-à-dire de telle sorte que l'ellipse passant au fond des encoches 51 et indiquée en pointillés (et qui correspond à l'évidement elliptique 49 de l'anneau 47) n'est pas interrompue. De plus, les deux encoches 51 sont légèrement évasées vers l'extérieur afin de faciliter l'engagement du disque 48 dans l'anneau 47 et l'introduction des parties de l'anneau 47 situées aux extrémités de son grand axe dans les encoches 51 du disque 48, et afin de faciliter également les pivotements du disque 48 par rapport à l'anneau 47 autour de leur grand axe sensiblement commun. Cette possibilité de rotation combinée à l'élasticité des deux organes 47 et 48 permet de former, lorsque le disque 48 est pivoté par rapport à l'anneau 47 de sorte que ses deux moitiés sont chacune appliquée sensiblement contre une moitié correspondante de l'anneau 47, mais de part et d'autre de ce dernier, deux pinces dont chacune est formée par la coopération de la moitié du disque 48 et de la moitié de l'anneau 47 en regard l'une de l'autre, et dans lesquelles peuvent être retenus les côtés internes des éléments sous-épineux 27, comme cela est schématiquement représenté sur la figure 8.

La jonction postérieure entre les deux enveloppes hémi-thoraciques tronçoniques 22a et 22b est ainsi assurée par une articulation dorsale élastique 36, présentant l'avantage mécanique de maintenir chacun des éléments à joindre pincé à l'intérieur de deux lèvres constituées chacune par la moitié d'un organe élastique elliptique, dont la forme s'accommode des déformations différentielles dans tous les sens des deux enveloppes hémi-thoraciques, l'orifice elliptique central 50 du disque 48 permettant de plus le réglage du jeu élastique de l'articulation 36, dont l'épaisseur, au niveau de la jonction, est limitée.

Bien entendu, il est possible d'utiliser une articulation élastique de structure différente de celle décrite ci-dessus pour réaliser la jonction postérieure des enveloppes hémi-thoraciques.

L'appareil ainsi décrit comporte donc trois enveloppes composites et tronçoniques, dont l'une, inférieure, enserre le bassin dans son ensemble, et dont chacune des deux autres, supérieure, enserre un hémi-thorax, et ces enveloppes sont équipées de moyens élastiques et reliées par des moyens élastiques dont l'un au moins est sollicité et chargé par tout mouvement volontaire du tronc du porteur, et tend ensuite par réaction, à rappeler le tronc dans sa position naturelle, en station verticale. En effet, les leviers-ressorts 45 encaissent les mouvements de flexion du tronc vers l'avant comme vers l'arrière ainsi que les mouvements de torsion du tronc d'un côté comme de l'autre, et se chargent comme des accumulateurs d'énergie qui tendent à se décharger en rappelant le tronc en mouvement inverse. Les tendeurs 46 fonctionnent de manière analogue comme des freins chargés par un mouvement vers l'avant et s'opposant à la chute vers l'avant du thorax, et l'un deux est bandé lors d'une torsion du tronc et tend à rappeler en torsion dans le sens contraire. De plus, le tendeur 37 est chargé lors d'un mouvement de l'épaule correspondante vers l'arrière, et il tend ensuite à rappeler l'épaule et à la faire descendre, notamment en réponse aux mouvements de vis, qui combinent une rotation du tronc avec une élévation d'une épaule et un abaissement de l'autre. Enfin, le tendeur 38 tend à serrer la cage thoracique et est chargé par un mouvement vers l'arrière. Les effets combinés de ces différents moyens élastiques ainsi que des bandes élastiques sur lesquelles sont implantées les plaques semi-rigides, en réponse à un quelconque mouvement volontaire du tronc du porteur, permettent de réactiver suffisamment les moteurs pelvien et scapulaires pour rendre possible l'utilisation des appareillages des membres supérieurs et inférieurs décrits dans les deux demandes de brevet précitées.

Les différents composants de l'appareillage selon l'invention, qui est de préférence noyé dans un scaphandre, entre deux couches de tissu par exemple, ou intégré dans un vêtement ré-éducatif, peuvent être règlés en fonction des troubles de la statique ou d'un déséquilibre antéropostérieur et/ou latéral et/ou en torsion du porteur. L'appareillage permet d'assurer le maintien dynamique, en cas de paralysie haute de la colonne vertébrale, et facilite la transmission des tensions et mouvements sur le moteur pelvien. L'appareillage selon l'invention permet donc d'apporter une certaine correction aux troubles du positionnement, une certaine amplification ou substitution aux troubles de la motricité et des informations de déplacement et/ou de positionnement en cas de trouble de la sensibilité.

Il est bien entendu que le mode de réalisation ci-dessus décrit n'est aucunement limitatif et pourra donner lieu à toutes modifications désirables sans sortir pour cela du cadre de l'invention.

## Revendications

1. Appareillage externe du tronc, caractérisé par le fait qu'il comprend deux structures composites,
- la première structure étant une structure inférieure, de forme générale externe tronconique, destinée à enserrer le bassin et l'abdomen et constituée par un corset pelvien (1) s'étendant sensiblement du périmètre bi-trochantérien en bas jusqu'à, d'une part, sensiblement l'ombilic, en haut et en avant, et, d'autre part, la moitié inférieure de la colonne lombaire, en haut et en arrière, ledit corset (1) comportant:
  a) une pluralité d'éléments semi-rigides (3, 4, 5, 6) reliés par des organes souples et flexibles (13, 14, 15, 16);
  b) des moyens élastiques régulateurs (17, 20) reliant chacune des deux parties (18) du corset (1) situées au droit des deux épines iliaques antéro-supérieures à la partie (19) du corset (1) située au droit de l'attache sacro-iliaque homo-latérale, d'une part, et à la partie (21) du corset (1) située au droit de l'ischion homolatéral, d'autre part;
- la deuxième structure composite étant une structure supérieure comportant deux enveloppes hémi-thoraciques symétriques (22a, 22b), qui ont chacune une forme générale externe tronconique et entourent chacune un hémi-thorax, les deux enveloppes hémi-thoraciques (22a, 22b) présentant une partie sterno-xyphoïdienne commune (23-28) et comportant chacune deux sous-ensembles, dont l'un est un sous-ensemble scapulaire et l'autre un sous-ensemble thoracique inféro-latéral, chacun de ces deux sous-ensembles étant constitué d'une pluralité d'éléments semi-rigides (25, 26, 27; 28, 29, 30) reliés par des organes souples et flexibles (31, 32; 33, 34, 35), les régions du creux axillaire et du sein restant libres, la structure supérieure comportant également:
  a) une articulation élastique dorsale (36), centrée au sommet de la colonne dorsale et reliant l'une à l'autre, et de manière détachable, les deux enveloppes hémi-thoraciques (22a, 22b);
  b) des moyens élastiques régulateurs (37, 38) reliant, sur chacune des deux enveloppes hémi-thoraciques (22a, 22b), d'une part, les éléments semi-rigides (25, 26, 27) du sous-ensemble scapulaire, et d'autre part, les éléments semi-rigides (28, 29, 30) du sous-ensemble thoracique;
  les deux structures composites précitées étant reliées l'une à l'autre par des moyens de liaison et de règlage de l'équilibre dynamique de l'appareillage, qui comprennent:
  a) deux leviers-ressorts (45) antérieurs, tendus chacun entre l'une des deux parties (18) du corset (1) située au droit des épines iliaques antéro-supérieures et la portion homolatérale de la partie sterno-xyphoïdienne commune (23-28), lesdits leviers-ressorts étant capables d'encaisser les déformations en flexion et/ou en torsion du tronc et de régler les mouvements relatifs de la colonne vertébrale, du bassin et de l'ensemble thoracique;
  b) deux tendeurs latéro-postérieurs (46) reliant chacun la zone sous-épineuse (27) du sous-ensemble scapulaire de l'une des deux enveloppes hémi-thoraciques (22a, 22b) à la partie (18) du corset (1) située au droit de l'épine iliaque du côté opposé, lesdits tendeurs étant destinés à freiner une chute du thorax vers l'avant.

2. Appareillage selon la revendication 1, caractérisé par le fait que le corset comporte quatre éléments semi-rigides (3, 4, 5, 6), les deux premiers éléments (3, 4) étant sensiblement égaux et ayant approximativement la forme d'un triangle équilatéral dont le côté est sensiblement égal au demi-périmètre de la taille, l'un desdits premiers éléments étant un élément postérieur (3), qui se place sur la région sacro-coccygienne, de façon que son côté inférieur soit disposé selon le demi-périmètre postérieur bi-trochantérien et l'autre desdits premiers éléments étant un élément antérieur (4), qui se place sur le symphyse pubienne, de façon que son côté supérieur soit disposé selon le demi-périmètre antérieur de la taille, les deux autres éléments semi-rigides du corset (1) étant des éléments sensiblement égaux, de forme sensiblement trapézoïdale, situés chacun en position intermédiaire entre les premiers éléments antérieur (4) et postérieur (3), les côtés supérieurs de ces éléments trapézoïdaux etant disposés au niveau de la taille et étant sensiblement égaux au demi-périmètre de ladite taille, ces deux éléments intermédiaires (5, 6) se plaçant symétriquement de chaque côté sur les deux os iliaques.

3. Appareillage selon la revendication 2, caractérisé par le fait que l'élément semi-rigide antérieur (4) du corset (1) est subdivisé, le long d'une ligne verticale médiane, en deux moitiés (4a, 4b) portant des moyens complémentaires d'un mécanisme d'ouverture et de fermeture (9) des deux moitiés l'une par rapport à l'autre, afin de permettre l'ouverture et la fermeture antérieures du corset (1).

4. Appareillage selon l'une des revendications 2 ou 3, caractérisé par le fait que les deux sommets adjacents audit côté horizontal des éléments triangulaires antérieur (4) et postérieur (3) du corset (1) sont coupés, afin de délimiter des espaces libres (10, 11, 7, 8) autorisant les mouvements du bassin.

5. Appareillage selon l'une des revendications 2 à 4, caractérisé par le fait que chacun des quatre éléments (3, 4, 5, 6) semi-rigides du corset (1) est relié à chacun des deux éléments qui lui sont adjacents par une bande de jointure élastique (13, 14, 15, 16) qui occupe également les espaces (7, 8, 10, 11) éventuellement libérés par la coupe des sommets des éléments postérieur (3) et antérieur (4).

6. Appareillage selon la revendication 5, caractérisé par le fait que les parties (18) du corset (1), sur lesquelles sont implantés les leviers-ressorts antérieurs (45), les tendeurs latéro-postérieurs (46) et les moyens élastiques régulateurs (17, 20), aux niveaux des épines iliaques, sont situées sur les bandes de jointure élastique (14, 15) reliant l'élément antérieur (4) aux éléments intermédiaires (5, 6), ainsi que sur les bords adjacents de ces éléments (4, 5, 6).

7. Appareillage selon l'une des revendications 5 ou 6, caractérisé par le fait que les parties (19, 21) du corset (1) sur lesquelles sont implantés les moyens élastiques régulateurs (17, 20), aux niveaux des attaches sacro-iliaques et des ischions, sont situées sur les bandes de jointure élastique (13, 16) entre l'élément postérieur (3) et les éléments intermédiaires (5, 6), ainsi que sur les bords adjacents de ces éléments (3, 5, 6).

8. Appareillage selon l'une des revendications 5 à 7, caractérisé par le fait que les quatre éléments semi-rigides (3, 4, 5, 6) du corset (1) sont des plaques moulées en matière plastique implantées sur une bande élastique (2).

9. Appareillage selon l'une des revendications 2 à 8, caractérisé par le fait que les moyens élastiques régulateurs du corset (1) sont constitués par quatre bandes élastiques (17, 20), régulant l'écartement des éléments intermédiaires (5, 6) du corset (1), et dont les deux bandes inférieures (20) reliant les parties (18) au droit des épines iliaques aux parties (21) au droit des ischions, forment des freins limitant le jeu antérieur de l'élément postérieur (3) du corset (1) et s'opposant à la lordose, tandis que les deux bandes supérieures (17), reliant les parties (18) au droit des épines iliaques aux parties (19) au droit des attaches sacro-iliaques, forment des freins s'opposant à la cyphose.

10. Appareillage selon l'une des revendications 1 à 9, caractérisé par le fait que les deux tendeurs latéro-postérieurs (46) sont chacun constitués par une bande élastique.

11. Appareillage selon l'une des revendications 1 à 10, caractérisé par le fait que chacun des deux leviers-ressorts (45) est constitué par une lame métallique flexible capable d'encaisser et de restituer les contraintes de flexion et de torsion.

12. Appareillage selon l'une des revendications 1 à 11, caractérisé par le fait que chacun des deux sous-ensembles de la deuxième structure composite est constitué de trois éléments semi-rigides (25, 26, 27) reliés par des organes souples et flexibles (31, 32; 33, 34, 35), lesdits trois éléments semi-rigides étant, pour le sous-ensemble scapulaire, un élément pectoral (25), un élément trapézoïdal (26) et un élément sous-épineux (27) et, pour le sous-ensemble thoracique, un élément thoracique postérieur (30) se plaçant sur la région sous-scapulaire, un élément thoracique antérieur (29) se plaçant sur la région sous-mammaire, et un élément xiphoïdien (28) qui se place sur les cartilages costaux et appartient à la partie sterno-xyphoïdienne commune (23-28), les deux enveloppes hémi-thoraciques (22a, 22b) étant reliées entre elles par l'articulation élastique dorsale (36) au niveau des éléments sous-épineux (27) de leurs sous-ensembles scapulaires.

13. Appareillage selon la revendication 12, caractérisé par le fait que, sur le sous-ensemble scapulaire de chaque enveloppe hémi-thoracique (22a, 22b), les éléments pectoral (25), trapézoïdal (26) et sous-épineux (27) sont des triangles équilatéraux dont le côté est approximativement égal au bord externe de l'omoplate et à la hauteur d'un élément sternal (23) semi-rigide, de forme sensiblement rectangulaire, de la partie sterno-xyphoïdienne commune (23, 28), l'élément trapézoïdal (26) présentant un sommet dirigé vers l'épaule et le côté opposé tourné vers le cou, en étant relié par son côté postérieur au côté supérieur de l'élément sous-épineux (27) par une bande de jointure élastique (32) et en faisant avec lui un angle d'environ 15°, l'élément sous-épineux (27) étant relié par son côté interne à l'articulation élastique dorsale (36) et par son côté externe à une bande de jointure élastique (33) le rattachant à l'élément thoracique postérieur (30) du sous-ensemble thoracique homo-latéral, en étant fixé par son sommet inférieur à l'extrémité supérieure du tendeur latéro-postérieur (46) homo-latéral, l'élément trapézoïdal (26) étant également relié le long de son côté antérieur, et par une bande de jointure élastique (31) au côté externe de l'élément pectoral (25), en faisant avec lui un angle d'environ 15° et l'élément pectoral (25) ayant son côté supérieur tourné vers la clavicule et étant incliné d'environ 30° par rapport à l'élément sternal (23), à l'extrémité supérieure duquel l'élément pectoral (25) est relié par son sommet opposé à son côté externe.

14. Appareillage selon l'une des revendications 12 ou 13, caractérisé par le fait que, sur le sous-ensemble thoracique de chaque enveloppe hémi-thoracique (22a, 22b), qui est tendu entre l'élément sous-épineux (27) du sous-ensemble scapulaire homo-latéral et la partie sterno-xyphoïdienne commune (23 - 28), les éléments thoracique postérieur (30), thoracique antérieur (29) et xyphoïdien (28) sont des triangles équilatéraux présentant un sommet dirigé vers le bas, l'élément thoracique antérieur (29) étant relié d'une part le long de son côté interne au côté externe de l'élément xyphoïdien (28) par une bande de jointure élastique (35), et d'autre part le long de son côté externe au côté externe de l'élément thoracique postérieur (30) par une bande de jointure élastique (34), en faisant entre ces côtés respectivement des angles d'environ 60°.

15. Appareillage selon l'une des revendications 12 à 14, caractérisé par le fait que la partie sterno-xyphoïdienne commune (23 - 28) est constituée d'une seule pièce présentant l'élément sternal (23) et les deux éléments xyphoïdiens (28).

16. Appareillage selon la revendication 15, caracterise par le fait que chacun des deux éléments xyphoïdiens triangulaires (28) est relié par son sommet interne supérieur à l'extrémité inférieure de l'élément sternal (23).

17. Appareillage selon l'une des revendications 12 à 16, caractérisé par le fait que, pour chaque enveloppe hémi-thoracique (22a, 22b), les trois éléments (25, 26, 27) du sous-ensemble scapulaire et les trois éléments (28, 29, 30) du sous-ensemble thoracique sont formés par une chaîne de six plaques en triangle équilatéral moulées en matière plastique et implantées sur une bande élastique (24a, 24b).

18. Appareillage selon l'une des revendications 12 à 17, caractérisé par le fait que les deux leviers ressorts antérieurs (45) sont implantés par leur extrémité supérieure chacun sur l'élément xyphoïdien (28) homo-latéral correspondant.

19. Appareillage selon l'une des revendications 12 à 18, caractérisé par le fait que les moyens élastiques régulateurs (37) reliant les trois éléments semi-rigides (25, 26, 27) du sous-ensemble scapulaire sur chacune des deux enveloppes hémi-thoraciques (22a, 22b) sont constitués par un tendeur élastique (37) ancré par une extrémité (39) sur le sommet inférieur de l'élément pectoral (25), par l'autre extrémité (40) sur le sommet inférieur de l'élément sous-épineux (27), et passant par le centre de gravité de l'élément trapézoïdal (26).

20. Appareillage selon l'une des revendications 12 à 19, caractérisé par le fait que les moyens élastiques régulateurs reliant les trois éléments semi-rigides (28, 29, 30) du sous-ensemble thoracique sur chacune des deux enveloppes hémi-thoracique (22a, 22b) sont constitués par un tendeur élastique (38) ancré par une extrémité (42) sur le sommet inférieur de l'élément xyphoïdien (28), par l'autre extrémité sur le sommet inférieur de l'élément thoracique postérieur (30) et passant par le centre de gravité de l'élément thoracique antérieur (29).

21. Appareillage selon la revendication 14, caractérisé par le fait que l'articulation élastique dorsale (36) comprend deux organes élastiques de forme elliptique (47, 48) ayant un grand axe sensiblement égal à la hauteur de la colonne dorsale et un petit axe sensiblement égal au côté des éléments (28, 29, 30) en triangle équilatéral des sous-ensembles thoraciques, l'un (47) desdits organes ayant la forme d'un anneau elliptique avec un grand évidement central (49), tandis que l'autre (48) a la forme d'un disque elliptique percé d'un petit évidement central (50) et présente une encoche axiale (51) s'étendant de chacune des deux extrémités de son grand axe vers son centre, la profondeur de ladite encoche axiale étant légèrement supérieure à la largeur de l'anneau (47) aux niveaux des extrémités de son grand axe, le disque (48) étant engagé dans l'anneau (47) et monté pivotant par rapport à ce dernier en chevauchant par encoches (51) les extrémités du grand axe de l'anneau (47), de sorte que les côtés internes des éléments sous-épineux (27) sont retenus chacun dans une pince constituée par la coopération d'une moitié du disque (48) et d'une moitié de l'anneau (47).

22. Appareillage selon l'une des revendications 1 à 21, caractérisé par le fait que les structures inférieure (1) et supérieure (22a, 22b), les leviers-ressorts (45) et les tendeurs (46) sont noyés dans un scaphandre.

**Patentansprüche**

1. Äußere Stützvorrichtung für den Rumpf, <u>dadurch gekennzeichnet</u>, daß sie aus zwei Strukturen zusammengesetzt ist, nämlich
- einer ersten unteren Struktur, die der allgemeinen äußeren Rumpfform entspricht und dazu bestimmt ist, das Becken und den Unterleib zu umschließen und aus einem Beckenkorsett (1) besteht, das sich im wesentlichen unten vom Trochanterumfang einerseits nach oben und nach vorne im wesentlichen bis zum Bauchnabel und andererseits nach oben und nach hinten bis zur unteren Hälfte der Lendenwirbel erstreckt, wobei das Korsett (1)

a) eine Vielzahl halbstarrer Elemente (3, 4, 5, 6), die durch weiche, flexible Organe (13, 14, 15, 16) verbunden sind, und

b) regulierende elastische Mittel (17, 20) aufweist, die jeweils zwei Teile (18) des Korsetts (1), die sich über den beiden vorderen oberen Darmbeinstacheln befinden, mit dem Teil (19) des Korsetts (1), das über dem homolateralen Kreuzbeinband liegt einerseits und andererseits mit dem Teil (21) des Korsetts (1) verbindet, das über dem homolateralen Sitzbein liegt;

- einer zweiten oberen Struktur mit zwei halbbrustförmigen symmetrischen Schalen (22a, 22b), die beide der allgemeinen äußeren Rumpfform entsprechen und die jeweils den halben Brustkorb umschließen, wobei die beiden halbbrustförmigen Schalen (22a, 22b) ein gemeinsames sterno-xiphoides Teil (23 - 28) darstellen und jeweils zwei Untereinheiten aufweisen, wovon eine eine Schulterblatteinheit ist und die andere eine untere seitliche Brustkorbeinheit darstellt, wobei jede dieser Untereinheiten aus einer Vielzahl von halbstarren Elementen (25, 26, 27; 28, 29, 30) besteht, die durch weiche und flexible Organe (31, 32; 33, 34, 35) verbunden sind, wobei die Gebiete der Achselhöhle und der Brust freibleiben und die obere Struktur außerdem aufweist:

a) ein elastisches Rückengelenk (36), dessen Zentrum am Scheitel der Rückenwirbelsäule liegt, und das die beiden halbbrustförmigen Schalen (22a, 22b) lösbar miteinander verbindet;

b) elastische regulierbare Mittel (37, 38), welche auf jeder der beiden halbbrustförmigen Schalen (22a, 22b) einerseits die halbstarren Elemente (25, 26, 27) der Schulterblattuntereinheit und andererseits die halbstarren Elemente (28, 29, 30) der Brustkorbuntereinheit verbinden, wobei die beiden oben erwähnten Strukturen miteinander durch Verbindungsmittel und durch Mittel zur Regulierung des dynamischen Gleichgewichts der Vorrichtung verbunden sind, die folgendes aufweisen:

a) zwei vordere Federn (45), die jeweils zwischen einem der beiden Teile (18) des Korsetts (1), das sich über den oberen vorderen Darmbeinstacheln befindet, und dem homolateralen Abschnitt des gemeinsamen sterno-xiphoiden Teils (23 - 28) gespannt sind, wobei die besagten Federn in der Lage sind, die Verformung beim Beugen und/ oder Drehen des Rumpfes aufzufangen und die relativen Bewegungen der Wirbelsäule, des Beckens und der Brustkorbeinheit zu regulieren;

) zwei hintere seitliche Spannbänder (46), die jeweils die Zone unter dem Schulterblatt (27) der Schulterblattuntereinheit einer der beiden halbbrustförmigen Schalen (22a, 22b) mit dem Teil (18) des Korsetts (1) verbinden, das über dem Darmbeinstachel liegt, wobei diese Bänder dazu bestimmt sind, einen Brustkorbvorfall zu verhindern.

2. Vorrichtung nach Anspruch 1, dadurch gekennzeichnet, daß das Korsett vier halbstarre Elemente (3, 4, 5, 6) aufweist, wobei die zwei ersten Elemente (3, 4) annähernd gleich sind und ungefähr die Form eines gleichseitigen Dreiecks haben, dessen Seite ungefähr gleich dem halben Tailleumfang ist, wobei eines der erstgenannten Elemente ein hinteres Element (3) ist, das über der Region des Steißbeines liegt, derart, daß seine untere Seite sich über den hinteren halben Trochanterumfang erstreckt, und das andere der besagten ersten Elemente ein vorderes Element (4) ist, das über dem Schambeinknochen liegt, derart, daß sich seine obere Seite über den vorderen halben Umfang der Taille erstreckt, die zwei anderen halbstarren Elemente des Korsetts (1) annähernd gleiche und etwa trapezförmige Elemente sind, die jeweils eine Mittellage zwischen den ersten vorderen Elementen (4) und den hinteren Elementen (3) einnehmen, und wobei die oberen Seiten dieser trapezförmigen Elemente in der Höhe der Taille angebracht sind und etwa den halben Umfang der Taille haben, wobei die beiden Zwischenelemente (5, 6) symmetrisch auf jeder Seite über den beiden Darmbeinstacheln liegen.

3. Vorrichtung nach Anspruch 2, dadurch gekennzeichnet, daß das vordere halbstarre Element (4) des Korsetts (1) durch eine mittlere vertikale Linie der Länge nach unterteilt ist in zwei Hälften (4a, 4b), die sich ergänzende Mittel eines Öffnungs- und Verschließmechanismus (9) für die beiden Hälften aufweisen, um das Korsett (1) vorne öffnen und verschließen zu können.

4. Vorrichtung nach Anspruch 2 oder 3, dadurch gekennzeichnet, daß die beiden Spitzen, die an die horizontale Seite der dreieckigen hinteren und vorderen Elemente (3 bzw. 4) des Korsetts (1) angrenzen, abgeschnitten sind, so daß freie Beckenbewegungen erlaubende Räume (10, 11, 7, 8) begrenzt werden.

5. Vorrichtung nach einem der Ansprüche 2 bis 4, dadurch gekennzeichnet, daß jedes der vier halbstarren Elemente (3, 4, 5, 6) des Korsetts (1) mit beiden dazu benachbarten Elementen mit einem elastischen Verbindungsband (13, 14, 15, 16) verbunden ist, das auch die gegebenenfalls - durch Abschneiden der Spitzen der hinteren und vorderen Elemente (3 bzw. 4) freigewordenen Räume (7, 8, 10, 11) beansprucht.

6. Vorrichtung nach Anspruch 5, dadurch gekennzeichnet, daß sich die Teile (18) des Korsetts (1), an denen die vorderen Federn (45), die seitlichen hinteren Spannbänder (46) und die regulierenden elastischen Mittel (17, 20) angebracht sind, auf Höhe der Darmbeinstachel auf den elastischen Verbindungsbändern (14, 15), die das vordere Element (4) mit den Zwischenelementen (5, 6) verbinden, sowie auf den benachbarten Rändern dieser Elemente (4, 5, 6) befinden.

7. Vorrichtung nach Anspruch 5 oder 6, dadurch gekennzeichnet, daß sich die Teile (19, 20) des Korsetts (1), an denen die elastischen Reguliermittel (17, 20) angebracht sind, in Höhe der Kreuzbeinbänder und der Sitzbeine auf den elastischen Verbindungsbändern (13, 16) zwischen dem hinteren Element (3) und den Zwischenelementen (5, 6) sowie auf den benachbarten Rändern dieser Elemente (3, 5, 6) befinden.

8. Vorrichtung nach einem der Ansprüche 5 bis 7, dadurch gekennzeichnet, daß die vier halbstarren Elemente (3, 4, 5, 6) des Korsetts (1) aus Kunststoff geformte Platten sind, die auf einem elastischen Band (2) befestigt sind.

9. Vorrichtung nach einem der Ansprüche 2 bis 8, dadurch gekennzeichnet, daß die regulierenden elastischen Mittel des Korsetts (1) aus vier elastischen Bändern (17, 20) bestehen, welche das Auseinanderklaffen der Zwischenelemente (5, 6) des Korsetts (1) regulieren und von denen die beiden unteren Bänder (20) die Teile (18) über den Darmbeinstacheln mit den Teilen (21) über den Sitzbeinen verbinden, Verzögerungseinrichtungen darstellen, welche das vordere Spiel des hinteren Elements (3) des Korsetts (1) begrenzen und der Lordose entgegenwirken, während die beiden oberen Bänder (17), welche die Teile (18) über den Darmbeinstacheln mit den Teilen (19) über den Kreuzbeinbändern verbinden, Verzögerungseinrichtungen darstellen, die der Kyphose entgegenwirken.

10. Vorrichtung nach einem der Ansprüche 1 bis 9, dadurch gekennzeichnet, daß die beiden hinteren seitlichen Spannbänder (46) jeweils aus einem elastischen Band bestehen.

11. Vorrichtung nach einem der Ansprüche 1 bis 10, dadurch gekennzeichnet, daß jede der vorderen Federn (45) aus einem flexiblen Metallstreifen besteht, der die beim Beugen und Drehen auftretenden Belastungen aufnehmen und wieder abgeben kann.

12. Vorrichtung nach einem der Ansprüche 1 bis 11, dadurch gekennzeichnet, daß jede der beiden Untereinheiten der zweiten Struktur aus drei halbstarren Elementen (25, 26, 27) besteht, die durch weiche und flexible Organe (31, 32; 33, 34, 35) verbunden sind, wobei die drei halbstarren Elemente für die Schulterblattuntereinheit ein Brustelement (25), ein trapezförmiges Element (26) und ein Element (27) unter dem Schulterblatt sowie für die Brustkorbuntereinheit ein hinteres Brustkorbelement (30), das sich über den Bereich unterhalb des Schulterblatts erstreckt, ein vorderes Brustelement (29), das sich über den Bereich unterhalb der Brust erstreckt, und ein xiphoides Element (28), das sich über die Rippenknorpel erstreckt und zu dem gemeinsamen sterno-xiphoiden Teil (23 - 28) gehört, darstellen, wobei die beiden halbbrustförmigen Schalen (22a, 22b) durch ein dorsales elastisches Gelenk (36) in Höhe der Elemente (27) unter den Schulterblättern ihrer Schulterblattuntereinheiten miteinander verbunden sind.

13. Vorrichtung nach Anspruch 12, dadurch gekennzeichnet, daß auf der Schulterblattuntereinheit jeder halbbrustförmigen Schale (22a, 22b) das pektorale Element (25), das trapezförmige Element (26) und das unterhalb des Schulterblatts befindliche Element (27) gleichseitige Dreiecke sind, deren Seite etwa gleich ist am Außenrand des Schulterblatts und auf der Höhe eines halbstarren, im wesentlichen rechteckigen sternalen Elements des gemeinsamen sterno-xiphoiden Teils (23, 28), wobei das trapezförmige Element (26) eine Spitze besitzt, die zur Schulter gerichtet ist und die gegenüberliegende Seite zum Hals zeigt und seine hintere Seite mit der oberen Seite des unterhalb des Schulterblatts befindlichen Elements (27) durch ein elastisches Verbindungsband (32) verbunden ist und damit einen Winkel von etwa 15° bildet, wobei das unterhalb des Schulterblatts befindliche Element (27) an seiner Innenseite mit dem dorsalen elastischen Gelenk (36) und an seiner Außenseite mit einem elastischen Verbindungsband (33) verbunden ist, das es an das hintere Brustelement (30) der homolateralen Brustuntereinheit bindet, wodurch es mit seiner unteren Spitze mit dem oberen Ende des homolateralen hinteren seitlichen Spannbandes (46) verbunden ist, wobei das trapezförmige Element (26) auch an seiner vorderen Seite durch ein elastisches Verbindungsband (31) mit der äußeren Seite des pektoralen Elements (25) verbunden ist und damit einen Winkel von etwa 15° bildet, und wobei die obere Seite des pektoralen Elements (25) zur Klavicula gerichtet und um etwa 30° bezüglich des sternalen Elements (23) geneigt ist, mit dessen oberen Ende das pektorale Element (25) mit seiner Spitze verbunden ist, die seiner äußeren Seite gegenüberliegt.

14. Vorrichtung nach Anspruch 12 oder 13, dadurch gekennzeichnet, daß auf der Brustkorbuntereinheit jeder halbbrustförmigen Schale (22a, 22b), die zwischen dem unter dem Schulterblatt befindlichen Element (27) der homo-lateralen Schulterblatteinheit und dem gemeinsamen sterno-xiphoiden Teil (23 - 28) gespannt ist, das hintere Brustkorbelement (30), das vordere Brustkorbelement (29) und das xiphoide Element (28) gleichseitige Dreiecke sind, welche eine nach unten gerichtete Spitze bilden, wobei das vordere Brustkorbelement (29) einerseits entlang seiner inneren Seite mit der äußeren Seite des xiphoiden Elements (28) durch ein elastisches Verbindungsband (35) und andererseits entlang seiner äußeren Seite mit der äußeren Seite des hinteren Brustkorbelements (30) durch ein elastisches Verbindungsband (34) verbunden ist, so daß zwischen den entsprechenden Seiten Winkel von etwa 60° gebildet werden.

15. Vorrichtung nach einem der Ansprüche 12 bis 14, dadurch gekennzeichnet, daß das gemeinsame sterno-xiphoide Teil (23 - 28) aus einem einzigen Stück besteht, das das sternale Element (23) und die xiphoiden Elemente (28) aufweist.

16. Vorrichtung nach Anspruch 15, dadurch gekennzeichnet, daß jedes der beiden dreieckigen xiphoiden Elemente (28) mit seiner oberen inneren Spitze mit dem unteren Ende des sternalen Elements (23) verbunden ist.

17. Vorrichtung nach einem der Ansprüche 12 bis 16, dadurch gekennzeichnet, daß bei jeder halbbrustförmigen Schale (22a, 22b) die drei Elemente (25, 26, 27) der Schulterblattuntereinheit und die drei Elemente (28, 29, 30) der Brustkorbuntereinheit aus einer Kette von sechs Platten gebildet sind, die die Form eines gleichseitigen Dreiecks besitzen, aus einem Kunststoffmaterial geformt sind und auf einem elastischen Band (24a, 24b) angebracht sind.

18. Vorrichtung nach einem der Ansprüche 12 bis 17, dadurch gekennzeichnet, daß die beiden vorderen Federn (45) jeweils mit ihrem oberen Ende auf dem entsprechenden homolateralen xiphoiden Element (28) befestigt sind.

19. Vorrichtung nach einem der Ansprüche 12 bis 18, dadurch gekennzeichnet, daß die regulierenden elastischen Mittel (37), welche die drei halbstarren Elemente (25, 26, 27) der Schulterblattuntereinheit auf jeder der beiden halbbrustförmigen Schalen (22a, 22b) verbinden, aus einem elastischen Spannband (37) bestehen, das mit einem Ende (39) an der unteren Spitze des pektoralen Elements (25) und mit dem anderen Ende (40) an der unteren Spitze des unterhalb des Schulterblatts befindlichen Elements (27) verankert ist und durch den Schwerpunkt des trapezförmigen Elements (26) verläuft.

20. Vorrichtung nach einem der Ansprüche 12 bis 19, dadurch gekennzeichnet, daß die regulierenden elastischen Mittel, welche die drei halbstarren Elemente (28, 29, 30) der Brustkorbuntereinheit mit jeder der beiden halbbrustförmigen Schalen (22a, 22b) verbinden, aus einem elastischen Spannband (38) bestehen, das mit einem Ende (42) an der unteren Spitze des xiphoiden Elements (28) und mit dem anderen Ende an der unteren Spitze des hinteren Brustkorbelements (30) verankert ist und durch den Schwerpunkt des vorderen Brustkorbelements (29) verläuft.

21. Vorrichtung nach Anspruch 14, dadurch gekennzeichnet, daß das dorsale elastische Element (36) zwei elliptische elastische Organe (47, 48) aufweist, die eine große Achse, welche im wesentlichen der Höhe der dorsalen Wirbelsäule entspricht, und eine kleine Achse besitzen, welche im wesentlichen der Seite der Elemente (28, 29, 30) der gleichseitigen Dreiecke der Brustkorbuntereinheiten entspricht, wobei eines (47) dieser Organe die Form eines elliptischen Rings mit einer großen zentralen Aussparung (49) besitzt, während das andere Organ (48) die Form einer elliptischen Scheibe mit einer kleinen zentralen Aussparung (50) besitzt und eine axiale Kerbe (51) aufweist, die sich von jedem der beiden Enden der großen Achse zum Zentrum erstrecken, wobei die Tiefe dieser axialen Kerbe geringfügig größer als die Breite des Ringes (47) in Höhe der Enden der großen Achse ist und wobei die Scheibe (48) im Eingriff mit dem Ring (47) ist und drehbar dazu befestigt ist, indem sie mit ihren Kerben (51) die Enden der großen Achse des Ringes (47) überlappen,

so daß die Innenseitender unter den Schulterblättern befindlichen Elemente (27) jeweils in einer Klemmvorrichtung gehalten sind, welche durch Zusammenwirken einer Hälfte der Scheibe (48) und einer Hälfte des Ringes (47) gebildet ist.

22. Vorrichtung nach einem der Ansprüche 1 bis 21, dadurch gekennzeichnet, daß die untere Struktur (1) und die obere Struktur (22a, 22b), die Federn (45) und die Spannbänder (46) in einen Anzug eingebettet sind.

**Claims**

1. An external appliance for the trunk, characterised in that it comprises two composite structures,
- the first structure being a lower structure of a general external frustoconical shape, intended to enclose the pelvis and abdomen and constituted by a pelvic corset (1), extending substantially from the perimeter of the two trochanters below up to, on the one hand, substantially the umbilicus above and in front, and on the other hand, to the lower half of the lumbar column above and at the back, the said corset (1) comprising:
a) a plurality of semi-rigid elements (3, 4, 5, 6) connected by pliable and flexible members (13, 14, 15, 16);
b) elastic regulating means (17, 20) connecting each one of the two parts (18) of the corset (1) situated opposite the two antero-superior iliac spines to the part (19) of the corset (1) situated opposite the homolateral sacroiliac articulation on the one hand, and to the part (21) of the corset (1) situated opposite the homolateral ischium on the other hand;
the second composite structure being an upper structure comprising two symmetrical hemithoracic shells (22a, 22b). which each have a general external frustoconical shape and each surround one hemithorax, the two hemithoracic shells (22a, 22b) having a common sterno-xiphoid part (23-28) and each comprising two sub-assemblies, whereof one is a scapular sub-assembly and the other an infero-lateral thoracic sub-assembly, each one of these two sub-assemblies being constituted by a plurality of semi-rigid elements (25, 26, 27; 28, 29, 30), connected by pliable and flexible members (31, 32; 33, 34, 35), the regions of the axillary fossa and of the breast remaining free, the upper structure also comprising:
a) an elastic dorsal articulation (38) centred at the top of the dorsal column and joining one to the other, and in a detachable manner, the two hemithoracic shells (22a, 22b);

b) elastic regulating means (37, 38) joining on each one of the two hemithoracic shells (22a, 22b) on the one hand the semi-rigid elements (25, 26, 27) of the scapular sub-assembly and on the other hand, the semi-rigid elements (28, 29, 30) of the thoracic sub-assembly, the two above mentioned composite structures being connected to each other by connecting means and means for the adjustment of the dynamic equilibrium of the appliance which comprise:

a) two anterior spring levers (45), each stretched between one of the two parts (18) of the corset (1) situated opposite the antero-superior iliac spines and the homolateral portion of the common sterno-xiphoid portion (23-28), the said spring levers being capable of absorbing the flexural and/or torsional deformations of the trunk and of regulating the relative movements of the vertebral column, the pelvis and of the thoracic unit;

b) two latero-posterior tensioners (48) each connecting the infraspinous zone (27) of the scapular sub-assembly of one of the two hemithoracic shells (22a, 22b) to the part (18) of the corset (1) situated opposite the iliac spine on the opposite side, the said tensioners being intended to restrain sagging of the thorax towards the front.

2. An appliance according to Claim 1, characterised in that the corset comprises four semi-rigid elements (3, 4, 5, 6), the first two elements (3, 4) being substantially equal and having approximately the shape of an equilateral triangle whose side is substantially equal to half of the waist measurement, one of the said first elements being a posterior element (3) which is placed over the sacrococcygeal region, so that its lower side should be disposed along the posterior half perimeter of the two trochanters and the other of the said first elements being an anterior element (4) which is placed over the pubic symphysis so that its upper side should be disposed along the anterior half perimeter of the waist, the two other semi-rigid elements of the corset (1) being substantially equal elements, of a substantially trapezoidal shape, each situated in an intermediate position between the first anterior element (4) and posterior element (3), the upper sides of these trapezoidal elements being disposed at the level of the waist and being substantially equal to the half-perimeter of the said waist, these two intermediate elements (5, 6) being symmetrically placed on either side of the two iliac bones.

3. An appliance according to Claim 2, characterised in that the anterior semi-rigid element (4) of the corset (1) is subdivided along a vertical median line into two halves (4a, 4b) carrying complementary means of an opening and fastening mechanism (9) for the two halves, one in relation to the other, so as to allow the anterior opening and fastening of the corset (1).

4. An appliance according to one of Claims 2 or 3, characterised in that the two apices adjacent to the said horizontal side of the anterior (4), and posterior (3) triangular elements of the corset (1) are cut so as to delimit free spaces (10, 11, 7, 8) allowing pelvic movement.

5. An appliance according to one of Claims 2 to 4, characterised in that each one of the four semi-rigid elements (3, 4, 5, 6) of the corset (1) is connected to each one of the two elements adjacent thereto by an elastic articulation strap (13, 14, 15, 16) which also occupies the spaces (7, 8, 10, 11) possibly freed by the cut out of the apices of the posterior (3) and anterior (4) elements.

6. An appliance according to Claim 5, characterised in that the parts (18) of the corset (1) whereon there are fixed the anterior spring levers (45), the latero-posterior tensioners (46), and the elastic regulating means (17, 20) at the level of the iliac spines, are situated on elastic articulation straps (14, 15) connecting the anterior element (4) to the intermediate elements (5, 6) as well as to the adjacent edges of these elements (4, 5, 6).

7. An appliance according to one of Claims 5 or 6, characterised in that the parts (19, 21) of the corset (1) whereon there are fixed the elastic regulating means (17, 20) at the levels of the sacro-iliac articulations and the ischia, are situated on the elastic articulation straps (13, 16) between the posterior element (3) and the intermediate elements (5, 6) as well as on the adjacent edges of these elements (3, 5, 6).

8. An appliance according to one of Claims 5 to 7, characterised in that the four semi-rigid elements (3, 4, 5, 6) of the corset (1) are plates moulded from a plastic material fixed to an elastic strap (2).

9. An appliance according to one of Claims 2 to 8, characterised in that the elastic regulating means of the corset (1) are constituted by four elastic straps (17, 20), adjusting the interspacing of the intermediate elements (5, 6) of the corset (1), and the two lower straps (20) whereof joining the parts (18) opposite the iliac spines to the parts (21) opposite the ischia, form restraints limiting the anterior play of the posterior element (3) of the corset (1) and resisting lordosis whilst the two upper straps (17) connecting the parts (18) opposite the iliac spines to the parts (19) opposite the sacro-iliac articulations, form restraints resisting kyphosis.

10. An appliance according to one of Claims 1 to 9, characterised in that the two latero-posterior tensioners (46) are each constituted by an elastic strap.

11. An appliance according to one of Claims 1 to 10, characterised in that each of the two spring levers (45) is constituted by a flexible metallic strip capable of absorbing and restoring the flexural and torsional stresses.

12. An appliance according to one of Claims 1 to 11, characterised in that each one of the two sub-assemblies of the second composite structure is constituted by three semi-rigid elements (25, 26, 27) joined by pliable and flexible members (31, 32; 33, 34, 35), the said three semi-rigid elements being, as regards the scapular sub-assembly, a pectoral element (25) a trapezial element (26) and an infraspinous element (27), and as regards the thoracic sub-assembly, a thoracic posterior element (30) being placed over the infrascapular region, an anterior thoracic element (29) being placed over the inframammary region and a xiphoid element (28) which is placed over the costal cartilages and appertaining to the common sterno-xiphoid portion (23-28), the two hemi-thoracic shells (22a, 22b) being interconnected by the elastic dorsal articulation (36) at the level of the infraspinous elements (27) of their scapular sub-assemblies.

13. An appliance according to Claim 12, characterised in that the scapular sub-assembly of each hemi-thoracic shell (22a, 22b), the pectoral (25), trapezial (26) and infraspinous (27) elements are equilateral triangles whose side is approximately equal to the external edge of the scapula and to the height of a semi-rigid sternal element (23), of a substantially rectangular shape, of the common sterno-xiphoid portion (23-28), the trapezial element (26) having a top directed towards the shoulder and the opposite side turned towards the neck being connected by its posterior side to the upper side of the infraspinous element (27) by an elastic articulation strap (32) and forming therewith an angle of approximately 15°, the infraspinous element (27) being connected at its internal side to the elastic dorsal articulation (36) and at its external side to an elastic articulation strap (33) fastening it to the posterior thoracic element (30) of the homolateral thoracic sub-assembly, being fixed at its lower tip to the upper end of the homolateral lateroposterior tensioner (46), the trapezial element (26) also being joined along its anterior side and by an elastic articulation strap (31) to the external side of the pectoral element (25) forming therewith an angle of approximately 15° and the pectoral element (25) having its upper side turned towards the clavicle and being inclined by approximately 30° in relation to the sternal element (23), at the upper end whereof, the pectoral element (25) is joined at its top opposite its external side.

14. An appliance according to one of Claims 12 or 13, characterised in that on the thoracic sub-assembly of each hemithoracic shell (22a, 22b) which is stretched between the infraspinous element (27) of the homolateral scapular sub-assembly and the common sterno-xiphoid portion (23-28), the posterior thoracic (30), the anterior thoracic (29), and xiphoid (28), elements are equilateral triangles having one apex directed towards the bottom, the anterior thoracic element (29) being joined on the one hand along its internal side to the external side of the xiphoid element (28) by an elastic articulation strap (35) and, on the other hand, along its external side to the external side of the posterior thoracic element (30) by an elastic articulation strap (34), forming betwen these sides angles of approximately 60° respectively.

15. An appliance according to one of Claims 12 to 14, characterised in that the common sterno-xiphoid part (23-28) is constituted by a single component having the sternal element (23) and the two xiphoid elements (28).

16. An appliance according to Claim 15, characterised in that each one of the two triangular xiphoid elements (28) is connected at its upper internal tip to the lower end of the sternal element (23).

17. An appliance according to one of Claims 12 to 16, characterised in that for each hemithoracic shell (22a, 22b) the three elements (25, 26, 27) of the scapular sub-assembly and the three elements (28, 29, 30) of the thoracic sub-assembly are formed by a chain of six plates in the shape of equilateral triangles moulded from a plastic material and fixed to an elastic articulation strap (24a, 24b).

18. An appliance according to one of Claims 12 to 17, characterised in that the two anterior spring levers (45) are each fixed at their upper ends to the corresponding homolateral xiphoid element (28).

19. An appliance according to one of Claims 12 to 18, characterised in that the elastic regulating means (37) connecting the three semi-rigid elements (25, 26, 27) of the scapular sub-assembly on each one of the two hemithoracic shells (22a, 22b), are constituted by an elastic tensioner (37) anchored at one end (39) on the lower tip of the pectoral element (25), at its other end (40) on the lower tip of the infraspinous element (27) and passing through the centre of gravity of the trapezial element (26).

20. An appliance according to one of Claims 12 to 19, characterised in that the elastic regulating means connecting the three semi-rigid elements (28, 29, 30) of the thoracic sub-assembly on each one of the two hemithoracic shells (22a, 22b) are constituted by an elastic tensioner (38), anchored at one end (42) on the lower tip of the xiphoid element (28), at its other end on the lower tip of the posterior thoracic element (30) and passing through the centre of gravity of the anterior thoracic element (29).

21. An appliance according to Claim 14, characterised in that the elastic dorsal articulation (36) comprises two elliptically shaped elastic members (47, 48) having the major axis substantially equal to the height of the dorsal column and the minor axis substantially equal to the sides of the elements (28, 29, 30) with the shape of equilateral triangles of the thoracic sub-assemblies, one, (47) of the said members having the shape of an elliptical ring with a large central cut out (49), whilst the other, (48), has the shape of an elliptical disc pierced by a small central cut out (50) and having an axial slot (51) extending from each one of the two ends of its major axis towards its centre, the depth of the said axial slot slightly exceeding the width of the ring (47) at the level of the ends of its major axis, the disc (48)

29 **0 153 237** 30

being engaged in the ring (47) and mounted to pivot in relation to the latter, by straddling the ends of the major axis of the ring (47) with its slots (51), so that the internal sides of the infraspinous elements (27) are each retained in a grip constituted by the cooperation of one half of the disc (48) and of one half of the ring (47).

22. An appliance according to one of Claims 1 to 21, characterised in that the lower, (1), and upper structures (22a, 22b), the spring levers (45) and the tensioners (48) are embedded in a diving suit.

16

**0 153 237**

FIG. 1

FIG. 2

1

FIG. 3

FIG. 4

FIG. 5

FIG. 6

47

49

FIG. 7

51

48

50

51

FIG. 8

47    51    48

26                    26

27                    27

30                    30

29                    29

50

51